# EUROPEAN PATENT APPLICATION

(11) **EP 1 504 770 A1**
(43) Date of publication of application: **09.02.2005**
(21) Application number: 04251200.4
(22) Date of filing: 02.03.2004
(51) Int. Cl.: A61L 15/42, A61F 13/15

(54) **Attachment of superabsorbent materials to fibers using oil**

(30) Priority: 05.08.2003 US 635062
(71) Applicant: Weyerhaeuser Company, Federal Way, WA 98063-9777 (US)
(72) Inventor: Hamilton, Robert T., Seattle, WA 98107 (US); Ma, Kiet, Federal Way, WA 98023 (US); West, Hugh, Seattle, WA 98115 (US); Halabisky, Donald D., Tacoma, WA 98422 (US)
(74) Representative: Hayes, Adrian Chetwynd

(57) **Abstract**

Oil is used in absorbent structures to promote the retention of superabsorbent materials within the absorbent structures that are formed from fibers and the superabsorbent material. The oil is applied to either the fibers or the superabsorbent material or both prior to or during the formation of the components into an absorbent structure. Acquisition rates and fluid retention levels of the absorbent structures are described.

## Description

### FIELD OF THE INVENTION

The present invention relates to the attachment of superabsorbent material to fibers using oil, methods of achieving such attachment, and compositions for use in attaching superabsorbent materials to fibers using oil.

### BACKGROUND OF THE INVENTION

In the production of absorbent articles, such as diapers and incontinent devices, it is known to combine superabsorbent materials and cellulose fibers to form an absorbent core. The absorbent core receives fluid to be absorbed and retains the fluid. When superabsorbent materials are in the form of powder or small particles, it is a challenge to retain the superabsorbent material in the absorbent core which comprises a matrix of fibers, commonly cellulose fibers. Various methods of retaining superabsorbent material in an absorbent core have been described. For example, it has been proposed that the cellulose fibers be embedded within the surface of the superabsorbent material.

Another approach described in International Publication Nos. WO 94/04352 and WO 94/04351 assigned to the assignee of the present application provides polymeric or non-polymeric binders between the superabsorbent material and the fiber. The binder is described as binding the superabsorbent material to the fiber through hydrogen or coordinate covalent bonds. The noted international publications describe that the addition of small amounts of moisture to the particles or fibers is desirable to promote binding between the superabsorbent material and the fibers. The moisture is described as being provided naturally by the ambient environment such as when the relative humidity of the environment where the superabsorbent material, binder and fibers are combined is approximately 60% to 75%, or higher. In instances where it is determined that the moisture is necessary and the relative humidity is below a desired level, capital equipment can be used in order to humidify the manufacturing location where the superabsorbent material, binder and fibers are contacted. While humidifying the manufacturing location is effective, it requires a capital investment and increases the costs of production.

Retaining the superabsorbent material in an absorbent core over an extended period of time is another challenge that faces manufacturers. The retention of superabsorbent materials in the absorbent core may fail over time for a number of reasons such as vigorous handling of the absorbent core which results in dislodgment of the superabsorbent material or a reduction in the moisture content of the absorbent core.

Diapers including an absorbent core of superabsorbent material and cellulose fibers are typically manufactured by a process that combines cellulose fibers and superabsorbent material. In such a process, rolls or bales of cellulose fibers without superabsorbent material are fiberized by a fiberizing apparatus such as a hammermill. These fiberized cellulose fibers are entrained in air and superabsorbent material is introduced to the air entrained fibers. The air entrained combination of cellulose fibers and superabsorbent material is delivered to an air lay device such as a pad former, which draws the fibers and superabsorbent material onto a screen and forms the fibers and superabsorbent material into a particular shape. These formed pads are then removed from the pad former for further processing. In order to maximize the utilization of the superabsorbent material and minimize the amount of superabsorbent material lost during manufacturing, diaper manufacturers desire that superabsorbent material be attached to the fiber during the time that the fiber and superabsorbent material are air entrained together.

With this background, the present inventors have worked to address the challenges above and have developed compositions and methods that employ oil to assist in the retention of superabsorbent materials in an absorbent article.

### SUMMARY OF THE INVENTION

The present invention provides fibers and/or superabsorbent materials treated with oil that are useful in absorbent cores formed from the treated fibers and/or treated superabsorbent materials and untreated fibers and/or untreated superabsorbent materials. The compositions of the present invention can be formed into absorbent articles for absorbing fluids such as aqueous fluids like urine or blood. The compositions are useful in methods for retaining superabsorbent materials in webs or masses of fibers commonly used as absorbent structures in absorbent articles such as diapers, incontinent devices and feminine hygiene products. The methods provide absorbent structures that are able to retain superabsorbent materials at a level that manufacturers of absorbent articles should find desirable. Surprisingly, when oil is used in accordance with the present invention, despite the hydrophobicity of the oil, absorbent articles that include fibers or superabsorbent material treated with the oil acquire and retain fluids at a rate and in an amount that manufacturers and customers of the absorbent articles should find desirable. Other advantages of using oil as described below in more detail include the ability to produce an absorbent article that retains superabsorbent material irrespective of the humidity present when the absorbent article is manufactured or the humidity that the manufactured absorbent article is exposed to over time.

In one aspect, the present invention relates to articles for absorbing aqueous fluids that include cellulose fibers and superabsorbent material wherein the cellulose fibers or superabsorbent material are treated with an oil. The invention also relates to the cellulose fibers that have been treated with an oil and superabsorbent materials that have been treated with an oil, wherein the oil has a melting point below the temperature at which the oil is applied to the fibers or superabsorbent material.

In another aspect, the present invention relates to methods of retaining superabsorbent materials within a web of cellulose fibers. In one embodiment, the method provides cellulose fibers having an oil applied thereto, wherein the oil has a melting point below the temperature at which the oil is applied to the fibers. The oil treated cellulose fibers are then contacted with a superabsorbent material. In another embodiment of this aspect of the invention, the method provides superabsorbent materials having an oil applied thereto, wherein the oil has a melting point below the temperature at which the oil is applied to the superabsorbent material. The oil treated superabsorbent material is then contacted with cellulose fibers.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 is a graph illustrating the results of testing to determine the levels of superabsorbent material retained within various absorbent structures;
FIGURE 2 is a graph illustrating the acquisition times for the absorbent structures of FIGURE 1;
FIGURE 3 is a graph illustrating the amount of fluid acquired by the absorbent structures of FIGURE 1; and
FIGURE 4 is an illustration of the device used in the saddle wicking test.

### DETAILED DESCRIPTION OF THE PREFERRED EMBODIMENT

As used herein, the term "fiber" refers to natural or synthetic fibers. Such fibers may be physically pretreated, e.g., by subjecting the fibers to steam, or chemically treated, e.g., by crosslinking the fibers. The fibers may also be twisted or crimped as desired.

A particular type of fiber are cellulose fibers. A particular example of a cellulose fiber is wood pulp fiber. Wood pulp fibers can be hardwood pulp fibers or softwood pulp fibers. The pulp fibers may be chemical, thermomechanical, chemithermomechanical or combinations thereof. Such wood pulp fibers can be obtained from well known chemical processes such as the kraft or sulfite processes. Other cellulose fibers include lyocell, linen, chopped silk fibers, bagasse, hemp, jute, rice, wheat, bamboo, corn, sisal, cotton, flax, kenaf, peat moss, and mixtures thereof. When the fibers are cellulose fibers, they may be pretreated with chemicals to result in lignin or cellulose-rich fiber surfaces. In addition, the fibers may be bleached.

Examples of synthetic fibers include acrylic, polyester, carboxylated polyolefin, and polyamine fibers.

As used herein, the term "superabsorbent material" refers to polymers that swell on exposure to water and form a hydrated gel (hydrogel) by absorbing large amounts of water. Superabsorbent materials exhibit the ability to absorb large quantities of liquid, i.e., in excess of 10 to 15 parts of liquid per part thereof. These superabsorbent materials generally fall into three classes, namely starch graft copolymers, crosslinked carboxymethylcellulose derivatives and modified hydrophilic polyacrylates. Examples of such absorbent polymers are hydrolyzed starch-acrylonitrile graft copolymer, a neutralized starch-acrylic acid graft copolymer, a saponified acrylic acid ester-vinyl acetate copolymer, a hydrolyzed acrylonitrile copolymer or acrylamide copolymer, a modified crosslinked polyvinyl alcohol, a neutralized self-crosslinking polyacrylic acid, a crosslinked polyacrylate salt, carboxylated cellulose, and a neutralized crosslinked isobutylene-maleic anhydride copolymer.

Superabsorbent particles are available commercially, for example starch graft polyacrylate hydrogel fines (IM 1000F) from Hoechst-Celanese of Portsmouth, VA, or larger particles such as granules. Other superabsorbent particles are marketed under the trademarks SANWET (supplied by Sanyo Kasei Kogyo Kabushiki Kaisha), SUMIKA GEL (supplied by Sumitomo Kagaku Kabushiki Kaisha and which is emulsion polymerized and spherical as opposed to solution polymerized ground particles), FAVOR (supplied by Stockhausen of Greensboro, North Carolina), and NORSOCRYL (supplied by Atochem).

The term oil as used generally applies to a wide range of substances. Oils may be derived from animals or from plant seeds or nuts, and these types of oils tend to be chemically identical with fats, with the only difference being one of consistency at room temperature. Animal and plant oils are composed largely of triglycerides of the fatty acids, oleic, palmitic, stearic, and linolenic acid. Oils may also be derived from petroleum sources. Petroleum-based oils generally include a mixture of hydrocarbons. As used herein, the term "oil" refers to oils that have melting points below the temperature at which the oil is applied to the fibers or superabsorbent material as described below in more detail. Such temperature will generally be below 25°C, but could be higher. If the melting point of the oil is greater than the ambient temperature at which the oil is applied to the fibers or superabsorbent material, the oil can be heated to liquefy it. This ensures that the oils remain liquid during their application to the fibers or superabsorbent material. Oils useful in the present invention should also have a vapor pressure sufficiently low to prevent evaporation either during their application or during use.

The oil should not penetrate the walls of the fibers so rapidly that it becomes unavailable to retain the superabsorbent material when the superabsorbent material is contacted with the oil treated fibers. The oil preferably resides on the surface of the fibers during the useful life of the absorbent article made from the fibers, oil and superabsorbent material. To that end, oils of higher molecular weight penetrate the fiber wall more slowly than oils of a lower molecular weight.

With certain oils, particularly those that include unsaturated components such as soy bean oil, it has been observed that while functioning like other oils to promote retention of superabsorbent material within an absorbent structure comprising fibers and a superabsorbent material, after extended exposure to air it has been observed that soybean oil crosslinks and solidifies. The solidified soybean oil increases the hydrophobicity of the fibers to which it has been applied. For soybean oil and other oils that might exhibit such crosslinking, crosslinking inhibitors or stabilizers such as tert-butylhydroxyanisole (BHA, 2- or 3-tert-butyl-4-methoxy-phenol); butylhydroxytoluene (BHT, 2,6-di-tert-butyl-4-methyl-phenol); ascorbic acid (vitamin C); ascorbyl-palmitate; tocopherol (vitamin E); carnosic acid; and sesamin can be used to inhibit such crosslinking.

Examples of "oils" as that term is used herein include fats and their component fatty acids. As described above, fats are naturally occurring esters of long chain carboxylic acids and the triol glycerol. These esters are also referred to as triglycerides. The hydrolysis of fats yields glycerol and three component carboxylic acids. These straight chain carboxylic acids which may be obtained from the hydrolysis of fats are called fatty acids and include one carboxylic acid group. Fatty acids may be saturated or unsaturated. The most common saturated fatty acids are lauric acid, myristic acid, palmitic acid, and stearic acid. Other fatty acids include oleic acid, linoleic acid, and linolenic acid. Generally, the melting point of a fat depends on the amount of unsaturation in the fatty acids. Fats with a preponderance of unsaturated fatty acids generally have melting points below about 25°C. Specific examples of oils as that term is used herein include soybean oil, cottonseed oil, linseed oil, tung oil, castor oil, coconut oil, olive oil, canola oil, safflower oil, corn oil or jojoba oil. Jojoba oil is a light yellow liquid at room temperature that is not technically an oil or fat, but rather is a wax. A wax is an ester of fatty acids with long chain monohydric alcohols. The term oil as used herein is intended to include jojoba oil and other waxes that are liquid at temperatures that they are applied to fibers or superabsorbent materials in accordance with the present invention. It should be understood that the foregoing is a list of exemplary oils and the present invention is not necessarily limited to the foregoing oils. It should be understood that use of the term "oil" in this application refers not only to the oil itself comprising a mixture of various fat and fatty acid components, but also includes the individual isolated fats, and the isolated fatty acids that result when the fats are hydrolyzed. For example, the term "oil" as used herein also refers to the fatty acids oleic, palmitic, stearic, and linolenic, that form the most common triglycerides in many oils derived from animals and plants and would be useful in accordance with the present invention to retain superabsorbent material in an absorbent structure comprising fibers and the superabsorbent material.

The term "oil" as used herein also refers to unsubstituted alkanes, alkenes, alkynes, cycloalkanes, cycloalkenes, cycloalkynes, aromatics, and mixtures thereof derived from petroleum or animal sources that have melting points below the temperature at which the oil is applied to the fibers or as described below in more detail, to the superabsorbent materail, e.g., about 25°C. Such oils are generally derived from petroleum sources, but may also be derived from animal sources. Oils of this type useful in the present invention should have vapor pressure sufficiently low to prevent evaporation of the oil during application or use. Specific examples of these types of oils include mineral oil, paraffin oil, hexadecane, squalane, and squalene.

As used herein, mineral oil is an example of a highly refined liquid petroleum derivative. Mineral oil is light, clear, colorless, and odorless and is also referred to as medicinal oil. Mineral oil is used medicinally as an internal lubricant and for the manufacture of salves and ointments.

Paraffin oil is an example of an oil that is either pressed or dry distilled from paraffin distillate obtained from the distillation of petroleum.

Squalane is an example of an alkane derived from animal sources, such as the sebum. Squalene is an example of an alkene; more specifically, a terpene derived from animal sources, such as the human sebum or shark liver oil. Squalene may also be isolated from oils derived from plants, such as olive oil, wheat germ oil, rice bran oil, and yeast.

As described below in more detail, the present inventors have observed that an absorbent structure formed from fibers or superabsorbent material treated with an oil retains superabsorbent material within the structure to a greater degree than the amount of superabsorbent material retained within an absorbent structure that includes superabsorbent material and fibers that have not been treated with an oil in accordance with the present invention.

The retention of superabsorbent material within an air laid absorbent structure that includes fibers and superabsorbent material can be evaluating using a Rotap test as described below.

A ten by ten centimeter pad cut from an absorbent structure that includes fibers and superabsorbent material is placed on the top screen of a stack of screens. The stack of screens from top to bottom includes an 8-mesh screen, a 20-mesh screen, and a 200-mesh screen. A collection pan is provided below the 200-mesh screen to catch material that passes through the three screens. A lid is placed atop the 8-mesh screen. The screen stack is placed in a Rotap device which rotates the stack while at the same time, hitting the lid with a hammer. Both the rotation and the hammering occur at a frequency of about 2 Hz. Unless indicated otherwise, the sample is processed for two minutes. At the end of two minutes, the 200-mesh screen has a mixture of superabsorbent material and fibers collected on it. The fibers are easily lifted off with tongs and the superabsorbent material is weighed. For longer testing times, the superabsorbent material on the 200-mesh screen can be weighed at intervals. Rotap machines are manufactured by W.S. Tyler, Incorporated.

The present inventors have also observed that surprisingly, the presence of the oil, which behaves as a hydrophobic material, does not significantly alter the ability of the absorbent structure to acquire and transport fluid. The ability of an absorbent structure to acquire and transport fluid can be evaluated using the saddle wick test described below.

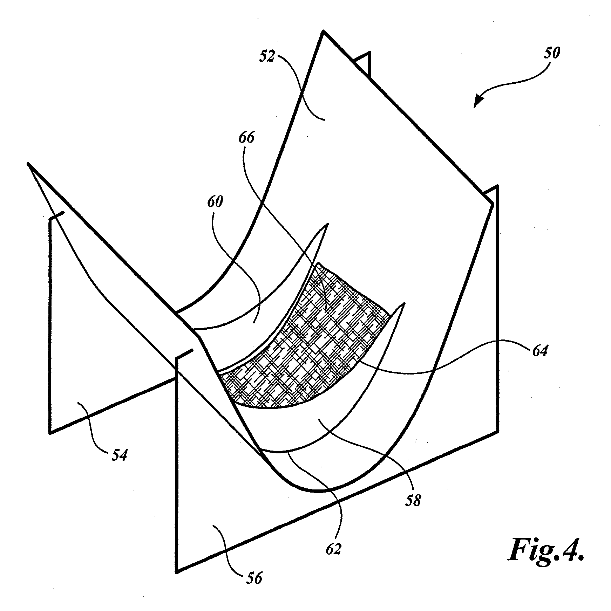

The saddle wicking test is used to show liquid distribution away from an initial dosing point into an absorbent structure. The test also measures how fast the liquid is absorbed for three separate doses into the absorbent structure. The test is carried out in the saddle wicking device which comprises the device illustrated in FIGURE 4.

Referring to FIGURE 4, the saddle wicking device 50 includes a u-shaped receptacle 52 which is supported by spaced apart left support plate 54 and right support plate 56. The support plates include a u-shaped cutout portion that forms a cradle for receiving and holding u-shaped receptacle in an upright position with its open end facing upward. Located in the trough of the u-shaped receptacle 52 are two spaced apart dams 58 and 60. Dams 58 and 60 include a lower edge 62 that mates with the bottom of the trough defined by u-shaped receptacle 52. The upper edge 64 of dams 58 and 60 is spaced above the lower edge so dams 58 and 60 form upward extending members that serve to block fluid which is dispensed between the dams from flowing past the dams.

The opened top of the u-shaped receptacle is approximately 23.2 cm across. The u-shaped receptacle is about 17.1 cm deep. The dams 58 and 60 are about 3.2 cm high at the bottom of the trough and extend up along the u-shaped receptacle to a location where the distance between one sidewall of the u-shaped receptacle to the other sidewall is about 14.0 cm. Dams 58 and 60 are spaced approximately 12.7 cm apart. The saddle wicking device can be manufactured from any rigid material such as plastic. As described below in more detail, sample 66 is positioned at the bottom of the u-shaped receptacle between dams 58 and 60.

The absorbent articles comprising superabsorbent materials and fibers having dimensions of about 30 cm long and 12.1 cm wide were divided into 3.8 cm wide sections moving to the left and the right of the center line of the sample. This results in the definition of sections F1, F2, F3, F4 to the right of the center line and B1, B2, B3, B4 to the left of the center line. These patterns are marked on the absorbent structure. The sample is then positioned and clamped in the saddle wicking device so that it maintains the same curvature as the device. The absorbent structure should not be bowed up in the center. Tape can be used if necessary on the bottom of the saddle wicking device to keep the absorbent structure from bowing up. An X is marked 2.5 cm to the right of the center line. This defines the insult point. A funnel is positioned 5 cm above the X. The funnel provides a flow rate ranging between 5-7 ml per second. A first insult of 100 ml of synthetic urine is dispensed from the funnel. The time is measured from the beginning of the insult to the time when the synthetic urine is fully absorbed into the absorbent structure. Full absorption is indicated when no liquid is seen in the creases and folds of the absorbent structure. The time is recorded for the first insult. The foregoing is repeated twice more using 50 ml of synthetic urine in the second and third insults. The times between starting of the insult and the time when the applied synthetic urine is fully absorbed is recorded for the second and third insult. The absorbent structure is then cut into the sections F1-F4 and B1-B4, as described above. Cutting of the absorbent structure should be completed within five minutes after the timer was stopped for the third insult. Each section is then weighed and the weight recorded. The cut sections are then placed on Plexiglas sheets and dried in an oven at 92-100°C for a minimum of 16 hours. The dried sections are then weighed and the weight recorded. The weight of synthetic urine absorbed in each respective zone is calculated by subtracting the dry weight from the wet weight of the particular sections.

In accordance with the present invention, the oil can be applied to the fiber or alternatively, the oil can be applied to the superabsorbent material. The oil-treated fiber or oil-treated superabsorbent material can then be combined with untreated superabsorbent material or untreated fiber, respectively, to form an absorbent article as described below in more detail. Oil-treated fibers and oil-treated superabsorbent materials may also be combined to form an absorbent article.

The particular way that oil is applied to the fibers is not critical. Examples of techniques for applying oil to the fibers include the use of a gravure-type roll coater to coat a web of the fibers. Alternatively, oil can be sprayed onto a web of the fibers or the fibers can be immersed in a bath of oil. The oil may also be added to the fibers as a web of the fibers is being broken up, such as in a hammermill. The amount of oil applied to the fibers should be sufficient to achieve the retention of superabsorbent material in accordance with the present invention, but not so much as to have a significant adverse affect on the fluid absorption properties of the fibers, such as the fluid acquisition rate or the amount of fluid absorbed by a web of the fibers. Manufacturers of absorbent articles that include absorbent structures containing the oil-treated fibers or oil-treated superabsorbent materials of the present invention desire that the fluid absorption properties of such structures be similar to the fluid absorption properties of the absorbent structures that the manufacturer is considering replacing. Ideally, the absorbent structures would exhibit fluid acquisition properties that are at least as desirable as the fluid acquisition properties of similar absorbent structures manufactured from untreated fibers. The amount of oil applied to the fibers should also not be so great that it adversely impacts the fiberization of the web of oil-treated fibers. Suitable amounts of oil applied to the fibers include about 0.5 wt. % to about 20 wt. % oil based on the weight of oven dried fibers. A narrower range is 1.0 wt. % to about 15 wt. % oil based on the weight of oven dried fibers and an even narrower range is 1.0 wt. % to about 10 wt. % oil based on the weight of oven dried fibers.

The form of the fibers to which the oil is applied can vary. If a roll coater is used, the fibers can be in the form of a sheet of fibers. The oil can be applied to a wet laid sheet of fibers having a basis weight of at least 350 grams per meter² and a density of at least about 400 kg/meter³.

The oil may be added neat, or it may be diluted with solvent that evaporates after application of the oil to the fibers. The solvent should not adversely affect the attachment of superabsorbent material to the fibers or the fluid acquisition and fluid retention properties of an absorbent article that contains the oil-treated fibers.

In the embodiment wherein the oil is applied to the superabsorbent material, again the method of application is not critical. The oil may be applied by spraying onto the superabsorbent material, or immersing the superabsorbent material in a bath of the oil.

The amount of oil applied to the superabsorbent material should be enough to result in attachment of the oil-treated superabsorbent material to fibers when combined with fibers, but should not be so great that it has a significant adverse affect on the fluid acquisition and fluid retention properties of a web of the fibers once the oil-treated SAP is combined therewith. In addition, the amount of oil employed should not be so great that it has a significant adverse affect on the absorbent properties of the superabsorbent material itself. Suitable amounts of oil applied to the superabsorbent material ranges from about 0.01 wt % to less than 10 wt. % oil based on the weight of superabsorbent material.

The oil-treated fibers and untreated superabsorbent material, the oil-treated superabsorbent material and untreated fibers, or the oil treated fibers and oil treated superabsorbent material can be combined and then formed into an absorbent structure in the following manner. The following description makes reference to fibers and superabsorbent material. It should be understood that the following description is applicable to the situation where oil-treated fibers are combined with untreated superabsorbent material, untreated fibers are combined with oil-treated superabsorbent material, or oil-treated fibers are combined with oil-treated superabsorbent material. Rolls or bales of fibers, without particles, are fiberized by a fiberizing device such as a hammermill. The individualized fibers are air entrained during which time the superabsorbent material can be added thereto. The air entrained fiberized fibers and superabsorbent material are then delivered to an air laying device, such as a pocket former, and formed into a desired shape. The formed pad is removed from the air laying device for further processing. The formed pads are in the form of a web or mass of fibers used as absorbent structures in absorbent articles such as the ones discussed above. The webs or masses of fibers have basis weights ranging from about 0.01 to about 0.05 g/cm², thicknesses ranging from about 0.3-2 millimeters and densities ranging from 0.15 to about 1 /cm³.

It should be understood that in an alternative embodiment, the oil can be applied to the combination of the fibers and superabsorbent material while they are air entrained together.

Combining the fibers and superabsorbent material can be carried out under a wide range of humidity conditions. In order to achieve retention of superabsorbent material within the absorbent article comprising the fibers and superabsorbent material, it is not required that any particular level of humidity be maintained when the two components are combined. Satisfactory retention can be achieved at relative humidity levels below about 70%, below about 60% and even below about 50%.

As illustrated in the examples that follow, the addition of oil to fibers or superabsorbent material in accordance with the present invention does not significantly affect the fluid acquisition and fluid retention properties of absorbent structures formed therefrom. Absorbent structures of the present invention acquire and retain fluids despite the presence of the hydrophobic oil. Absorbent structures produced in accordance with the present invention are soft and flexible.

The following examples are intended to illustrate certain embodiments of the present invention and are not intended to limit the scope of the present invention.

### EXAMPLE 1

### OIL TREATMENT OF FIBERS

Pulp sheets of southern pine fluff available from Weyerhaeuser Company under the designation NB416 with a starting moisture content of 6% by weight based on total sheet weight were coated in a Black Brothers gravure-type roll coater with mineral oil. The mineral oil was USP mineral oil purchased at a Rite Aid pharmacy carrying the label "Rite Aid Mineral Oil USP" and a CAS number 8020-83-5 and 8012-95-1, with alternative CAS numbers 39355-35-6, 79956-36-8 and 83046-05.3. The gravure coater applied a uniform coating of the mineral oil over one entire surface of the pulp sheet from where it was rapidly soaked up by the sheet. The mineral oil was applied to the pulp sheet at a rate of 10.5 parts by weight mineral oil to 100 parts of oven dried pulp. The treated sheet was stored in a plastic zippered bag for 24 hours at room temperature to allow the added oil to migrate within the sheet. The pulp sheets were then fiberized in a hammermill and combined with a superabsorbent material (available from Stockhausen under the designation SXM77) prior to delivery to a pocket former similar to pocket formers conventionally used in the manufacture of diapers and feminine hygiene products which was operated to produce a pad having a target fiber basis weight of 300 grams per square meter and a superabsorbent material basis weight of 200 grams per square meter. The pocket former was run at 18 pads per minute formation rate. The humidity ranged between 39% relative humidity at the beginning of the runs and 31% relative humidity at the end of the runs. The temperature started out at about 16.5°C and by the end of the runs had risen to about 22.5°C.

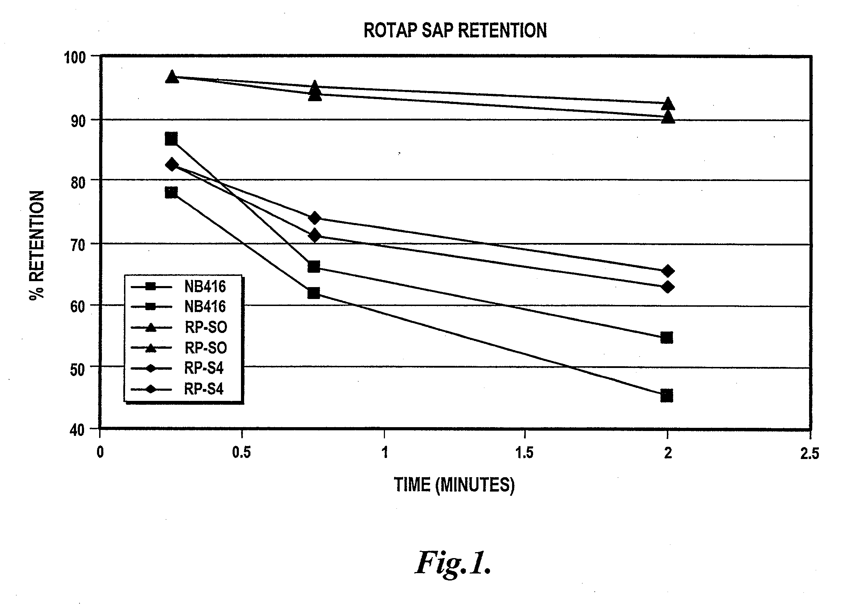

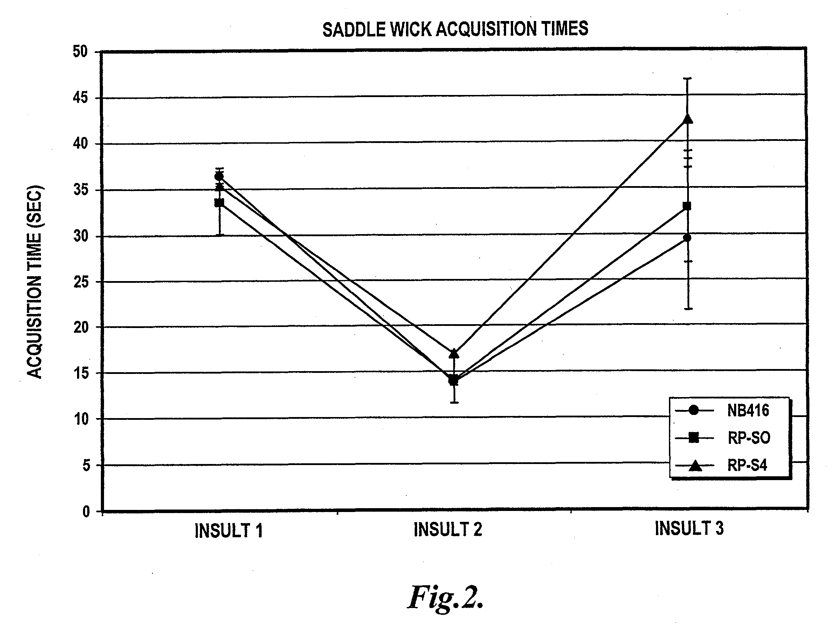

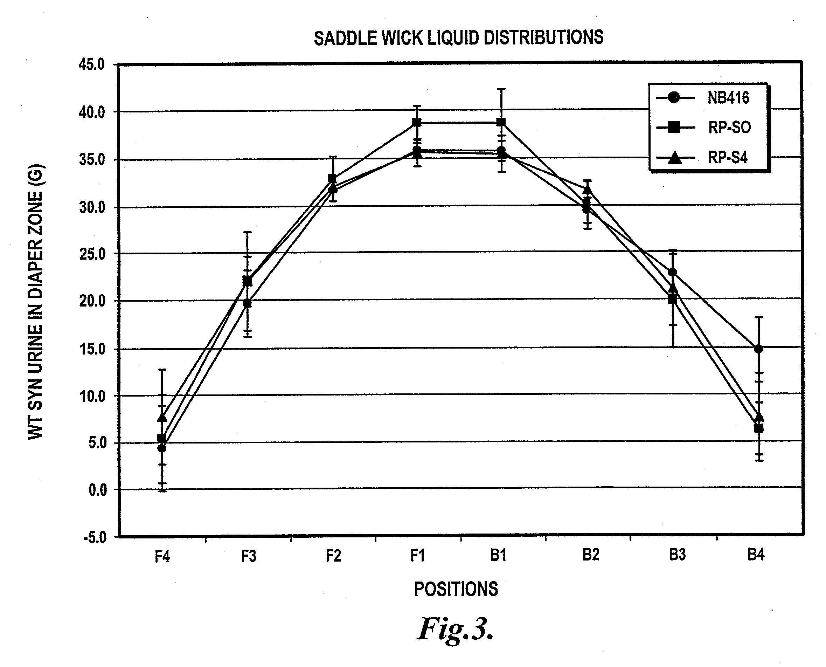

Some of the resulting pads were collected and subjected to Rotap testing. Other pads were densified to 0.25 cc/gm and subjected to saddle wick testing according to the techniques described above. In the Rotap testing, the pads were vibrated for two minutes. In the saddle wick testing, synthetic urine was employed using three insults of 100 ml, 50 ml, and 50 ml. The results are depicted in FIGURES 1, 2, and 3 as RP-SO.

### CONTROL EXAMPLE 1

For this control example, pads were prepared from the same type of fibers and superabsorbent material as Example 1 without mineral oil applied to the fibers. The same conditions as Example 1 were employed in preparing the pads of this control example. The collected pads were subjected to Rotap testing and saddle wick testing under the same conditions described in Example 1. The results are depicted in FIGURES 1, 2, and 3 as NB416.

### CONTROL EXAMPLE 2

In this control example, a commercial modified pulp available from Weyerhaeuser Company under the designation RP-S4 was used to form pads of such fibers and the same superabsorbent material described in Example 1. RP-S4 contains wood pulp fibers treated with a number of agents comprising molecules having at least two hydrogen bond or coordinate covalent bond forming functional groups or combinations of such functional groups which are contained on the fibers for the purpose of attaching superabsorbent material to the fibers. The pads were formed using the same conditions as Example 1. The collected pads were subjected to the Rotap testing and saddle wick testing under the same conditions described in Example 1. The results are illustrated in FIGURES 1, 2, and 3 as RP-S4.

The results illustrate that absorbent articles prepared from fibers treated with oil in accordance with the present invention and combined with superabsorbent material retain the superabsorbent material to the same level or greater than the pads prepared according to the control examples. Additionally, the results illustrate that absorbent articles formed in accordance with the present invention with oil present exhibit fluid acquisition rates and fluid retention levels similar to the pads prepared in accordance with the control example.

While the preferred embodiment of the invention has been illustrated and described, it will be appreciated that various changes can be made therein without departing from the spirit and scope of the invention.

## Claims

1. An article for absorbing an aqueous fluid, comprising:
cellulose fibers;
superabsorbent material; and
an oil applied to the cellulose fibers or the superabsorbent material.

2. The article of Claim 1 wherein the oil has a melting point below 25°C.

3. The article of Claim 1 or 2 wherein the oil comprises a triglyceride.

4. The article of Claim 1 or 2 wherein the oil is a fatty acid.

5. The article of Claim 1 or 2 wherein the oil is olive oil, soybean oil, safflower oil, cottonseed oil, linseed oil, tung oil, castor oil, coconut oil, canola oil, corn oil, or jojoba oil.

6. The article of Claim 1 or 2 wherein the oil is a saturated or unsaturated alkane, alkene, alkyne, cycloalkane, cycloalkene, cycloalkyne or combinations thereof.

7. The article of Claim 1 or 2 wherein the oil is petroleum derived.

8. The article of Claim 7 wherein the oil is mineral oil, hexadecane, squalane or squalene.

9. The article of any one of the preceding Claims wherein the oil is present on the fibers in an amount ranging from 0.5 to 20 wt.% based on the weight of oven-dried fibers.

10. The article of any one of the preceding Claims wherein the oil is present on the superabsorbent material in an amount ranging from 0.01 to less than 10 wt.% based on the weight of the superabsorbent material.

11. Treated fibers comprising:
cellulose fibers; and
an oil applied to the fibers, the oil having a melting point below the temperature at which the oil is applied to the fibers.

12. The treated fibers of Claim 11 wherein the cellulose fibers are wood pulp fibers.

13. Treated superabsorbent material comprising:
superabsorbent material; and
an oil applied to the superabsorbent material, the oil having a melting point below the temperature at which the oil is applied to the superabsorbent material.

14. The treated fibers of Claim 11 or 12 or the treated material of Claim 13 wherein the oil is petroleum derived.

15. The treated fibers of Claim 11 or 12 or the treated material of Claim 13 wherein the oil comprises a triglyceride.

16. A method for retaining superabsorbent material within a web of cellulose fibers comprising:
providing cellulose fibers having an oil applied thereto, the oil having a melting point below the temperature at which the oil is applied to the fibers; and
contacting a superabsorbent material with the cellulose fibers treated with an oil.

17. A method for retaining superabsorbent material within a web of cellulose fibers comprising:
providing superabsorbent material having an oil applied thereto, the oil having a melting point below the temperature at which the oil is applied to the superabsorbent material; and
contacting cellulose fibers with the superabsorbent material treated with an oil.
